# EUROPÄISCHE PATENTSCHRIFT

(11) **EP 3 639 023 B1**
(45) Veröffentlichungstag und Bekanntmachung des Hinweises auf die Patenterteilung: **04.05.2022**
(21) Anmeldenummer: 18756196.4
(22) Anmeldetag: 14.08.2018
(51) Int. Cl.: G01N 33/497, A61B 5/097

(54) **ATEMALKOHOLMESSUNG MIT BERÜHRUNGSLOSER PROBENABGABE**
BREATH ALCOHOL MEASUREMENT WITH CONTACTLESS SAMPLE PROVISION
MESURE DU TAUX D'ALCOOL DANS L'AIR EXPIRÉ AVEC PRÉLÈVEMENT D'ÉCHANTILLON SANS CONTACT

(30) Priorität: 25.08.2017 DE 102017008008
(43) Veröffentlichungstag der Anmeldung: 22.04.2020
(73) Patentinhaber: Dräger Safety AG & Co. KGaA, 23560 Lübeck (DE)
(72) Erfinder: REKOW, Jens, 23554 Lübeck (DE); MORLEY, Stefan, 23556 Lübeck (DE); BARTEN, Stefan, 23566 Lübeck (DE); STEMICH, Carsten, 23909 Ratzeburg (DE); RICHENBERGER, Michael, 23683 Scharbeutz (DE); SCHRÖTER, Sebastian, 23562 Lübeck (DE)
(74) Vertreter: Meyer-Gramann, Klaus Dieter
(86) Internationale Anmeldenummer: PCT/EP2018/072002
(87) Internationale Veröffentlichungsnummer: WO 2019/038131

(56) Entgegenhaltungen:
- WO-A1-2007/083350
- DE-U1- 8 225 425
- JP-A- 2013 156 169
- US-A- 4 852 583
- US-A1- 2011 283 770
- US-A1- 2013 231 871

## Beschreibung

Die Erfindung betrifft eine Vorrichtung zur Atemalkoholmessung mit einem Probensammler, in den von einem Probanden über einen Probeneinlass eine Atemprobe eingeleitet wird, ohne dass es zu einem Kontakt zwischen dem Mund des Probanden und dem Probeneinlass kommt. Die Atemalkoholmessvorrichtung verfügt über eine Sensoreinheit, der die zumindest teilweise durch den Sammlerauslass des Probensammlers ausströmende Atemprobe zuführbar ist und die auf der Grundlage eines in der Atemprobe enthaltenen Alkoholgehaltes ein Messsignal erzeugt. Ferner ist eine Steuer- und Auswerteeinheit vorgesehen, die unter Zugrundelegung des Messsignals den Alkoholgehalt der Atemprobe ermittelt und ein ergebnisspezifisches Signal an eine Ausgabeeinheit überträgt, die eine Information über den Atemalkoholgehalt der Atemprobe bereitstellt.

Die üblicherweise zum Einsatz kommenden Atemalkoholmessgeräte verfügen über Mundstücke, die vom Probanden während der Probenabgabe in den Mund genommen werden müssen. Zwischen den einzelnen Proben werden die Mundstücke aus hygienischen Gründen gewechselt oder es werden austauschbare Überzüge auf ein Mundstück aufgesetzt, um einen Kontakt des Mundstückes durch unterschiedliche Probanden zu verhindern.

Vor der eigentlichen Untersuchung der Atemprobe überwacht das Atemalkoholmessgerät, ob in das Mundstück gepustet wurde und ermittelt dann das in das Gerät eingeleitete Atemvolumen. Durch diese Verfahrensweise wird sichergestellt, dass lediglich unvermischte bzw. unverdünnte Atemluft zur Messung verwendet wird. Das Atemalkoholmessgerät entnimmt daraufhin aus diesem Atemluftstrom durch geeignete Vorrichtungen ein Teilvolumen, das dem eigentlichen Sensor der vielfach als elektrochemischer Sensor ausgeführt ist, zugeführt wird.

In vielen Anwendungen ist es allerdings sinnvoll, dass eine schnelle Messabfolge mit unterschiedlichen Probanden realisiert wird. Ein Wechsel der Mundstücke ist in diesem Fall aus Kosten- und Zeitgründen nicht erwünscht. Um derartige Messungen durchzuführen, beispielsweise um dem Probanden den Zugang zu einem bestimmten Sicherheitsbereich zu erlauben, pustet der Proband in Richtung einer Einlassöffnung des Probensammlers eines Atemalkoholmessgerätes, ohne dass es zu einem Kontakt zwischen dem Probensammler und dem Mund des Probanden kommt.

Ein derartiges Atemalkoholmessgerät, das eine berührungslose Probenabgabe ermöglicht, wird beispielsweise in der WO 2014 / 031071 A1 beschrieben. Das Atemalkoholmessgerät verfügt über einen Probensammler mit einer Probeneinlassöffnung, in die der Proband seine Atemprobe durch Hineinpusten abgibt. Innerhalb des Atemalkoholmessgerätes sind Elemente vorgesehen, um die abgegebene Probe zumindest kurzzeitig aufzustauen und einen Sensor mit Atemluft des Probanden zu versorgen. Überschüssige bzw. bereits für eine Messung verwendete Luft wird auf einer dem Probeneinlass gegenüberliegende Seite wieder aus dem Atemalkoholmessgerät hinaus geführt.

Bei kontaktlosen Messungen des Atemalkoholgehaltes werden üblicherweise trichterförmige Probeneinlässe verwendet, in die der Proband die Probe abzugeben hat. Problematisch bei den bekannten Probeneinlässen ist vielfach, dass in bestimmten Bereichen der Trichter vergleichsweise hohe Staudrücke initiiert werden und es teilweise zu unkontrollierten Rückströmungen kommen kann. Dies ist vor allem darauf zurückzuführen, dass die bekannten Geräte über einen geschlossenen Trichter mit einer großen Einlassöffnung und einem sich verjüngen Querschnitt verfügen. Derartige Rückströmungen aus dem Trichter gelangen oftmals auf das Gesicht der Messperson oder führen zu Verwirbelungen vor dem Gesicht, was von den Testpersonen regelmäßig als störend oder unhygienisch empfunden wird.

Außerdem entsteht durch eine derart geschlossene Bauweise des Trichters während des Einströmens des Gases ein Überdruck im Trichter, der dazu führt, dass die Durchspülung des Trichters unzureichend ist. Dies führt in vielen Fällen dann wiederum dazu, dass der Sensor nicht ausreichend mit Atemgas beaufschlagt wird und die korrekte Messung der Konzentration des Atemalkohols beeinträchtigt wird.

Ferner offenbart US2011/0283770 einen trichterförmigen Probensammler zur kontaktlosen Einleitung einer Atemprobe in ein optisches Atemanalysegerät.

Ausgehend von den bekannten Atemalkoholmessgeräten, bei denen die Abgabe von Atemproben ohne Berührung eines Mundstücks erfolgt, und den zuvor geschilderten Problemen liegt der Erfindung die Aufgabe zugrunde, ein derart gattungsgemäßes Gerät bereitzustellen, bei dem eine zuverlässige Durchströmung des Probeneinlasses gewährleistet wird. Rückströmungen innerhalb des Probensammlers sollen zuverlässig vermieden werden, insbesondere auch unabhängig von der Stärke und dem Einfallswinkel unter dem der Atemluftstrom in den Probeneinlass und den Probensammler eintritt. Der zum Einsatz kommende Probensammler soll weiterhin robust, leicht zu reinigen und verhältnismäßig einfach herzustellen sein. Weiterhin soll der anzugebende Probensammler die Möglichkeit bieten, austauschbar am Messgerät befestigt werden zu können, damit dieser bedarfsgerecht in bestimmten Zeitabständen ausgetauscht werden kann.

Die zuvor beschriebene Aufgabe wird mit einer Vorrichtung zur Atemalkoholmessung gemäß Anspruch 1 gelöst. Vorteilhafte Ausführungsformen der Erfindung sind Gegenstand der abhängigen Ansprüche und werden in der folgenden Beschreibung unter teilweiser Bezugnahme auf die Figuren näher erläutert.

Die Erfindung betrifft eine Vorrichtung zur Atemalkoholmessung mit einem Probensammler, in den vom Probanden über einen Probeneinlass eine Atemprobe einleitbar ist, ohne dass es zu einem Kontakt zwischen dem Probanden und dem Probeneinlass kommt.

Hierfür verfügt die Vorrichtung über die im Anspruch 1 aufgeführten Merkmale.

Die erfindungsgemäße Vorrichtung zeichnet sich somit dadurch aus, dass zwischen dem Probeneinlass und dem Auslass, bevorzugt zwischen dem Probeneinlass und einem Sammlerauslass des Probensammlers, zumindest eine zusätzliche Ausströmöffnung vorgesehen ist, durch die ein Teil der vom Probanden abgegebenen Atemprobe austritt. Durch diese technische Maßnahme wird eine geeignete Durchströmung des Hauptströmungskanals, insbesondere des Probensammlers, der trichterförmig ausgeführt ist, sichergestellt. Hierbei werden insbesondere die Entstehung zu hoher Staudrücke in einzelnen Bereichen des Probensammlers und darüber hinaus Rückströmungen zuverlässig vermieden. Das Vorsehen entsprechender Ausströmöffnungen stellt eine vergleichsweise einfach zu realisierende technische Maßnahme dar, die einen überaus überraschenden technischen Effekt bewirkt.

Z Der Probensammler ist mit einer Messeinheit verbunden, in der sich der Hauptströmungskanal fortsetzt und der Sensor angeordnet ist, und verfügt über einen Sammlerauslass, über den die in den Probensammler abgegebene Atemprobe in den Teil des Hauptströmungskanals, der sich in der Messeinheit befindet, eingeleitet wird. Z Erfindungsgemäß ist die wenigstens eine Ausströmöffnung zwischen dem Probeneinlass und dem Sammlerauslass angeordnet.

Ferner ist es auf vorteilhafte Weise denkbar, dass der Probensammler trichter- bzw. konusförmig ausgebildet ist. Mit einem derart, insbesondere trichterförmig ausgeführten Probensammler wird ein vergleichsweise großes Volumen des Atemgases dem strömungstechnisch nachgeschalteten, in der Messeinheit angeordneten Sensor in mit Umgebungsluft zumindest nahezu unverdünnter Form zugeführt. Vorzugsweise wird hierfür das aus dem Probensammler in den sich in der Messeinheit des Atemalkoholmessgerätes fortsetzenden Teil des Hauptströmungskanals eingeleitete Atemgas an einem Abzweig bzw. einer Ansaugöffnung kontinuierlich vorbeigeführt und zur Messung ein Teil des vorbeiströmenden Atemgasstroms in den zum Sensor führenden Messgaskanal gesaugt. Durch diese technische Maßnahme wird sichergestellt, dass zu einem geeigneten Zeitpunkt ein Messvolumen aus dem Hauptströmungskanal über den Abzweig aus dem vorbeiströmenden Volumenstrom entnommen werden kann, das repräsentativ für die sogenannte tiefe Lungenluft ist.

In einer speziellen Ausführungsform der Erfindung ist vorgesehen, dass der Probensammler wenigstens ein Befestigungselement aufweist, durch das der Probensammler mit der Messeinheit des Atemalkoholmessgeräts, in der der Sensor angeordnet ist, verbindbar ist. Der Hauptströmungskanal setzt sich auch in diesem Fall ausgehend vom Probensammler in der Messeinheit fort, wo im Bereich eines Abzweigs ein Messgaskanal zum Sensor führt. Durch das Vorsehen eines derartigen Befestigungselementes wird sichergestellt, dass der erfindungsgemäß ausgeführte Probensammler auf einfache Weise austauschbar an der Messeinheit eines Alkoholmessgeräts befestigt werden kann. Aufgrund dieser Maßnahme ist es vergleichsweise einfach möglich, einen vorzugsweise trichterförmigen Probensammler je nach Bedarf, insbesondere in bestimmten zeitlichen Abständen, zu wechseln. Bevorzugt findet der Austausch zerstörungsfrei und ohne Einsatz eines Werkzeugs statt. Alternativ ist es denkbar, die Befestigungselemente und/oder den Probesammler derart auszuführen, dass dieser nach einmaliger Entfernung von der Messeinheit eines Atemalkoholmessgeräts nicht erneut an dieser befestigt werden kann. Dies kann bspw. mit Hilfe von Sicherheitselementen sichergestellt werden, die nach dem einmaligen Entfernen des Probensammlers von der Messeinheit abbrechen oder auf andere Weise zerstört werden, sodass eine erneute Befestigung des Probensammlers an der Messeinheit ausgeschlossen ist.

Gemäß einer Weiterbildung ist es denkbar, dass die wenigstens eine zusätzliche Ausströmöffnung derart gestaltet und dimensioniert ist, dass nach Einleitung der Atemprobe in den Probensammler am Sensor zur Erfassung des Atemalkoholgehalts und/oder an einem Drucksensor, über den die Versorgung des Sensors zur Erfassung des Atemalkoholgehalts gesteuert wird, ein Druck erzeugt wird, der eine Differenz zum Druck in der Umgebung aufweist. Wesentlich für die wenigstens eine zusätzliche Ausströmöffnung ist, dass einerseits eine gute Durchströmung des bevorzugt trichterförmigen Probensammlers gewährleistet ist und andererseits ein kontinuierlicher Atemluftstrom erzeugt wird, der eine zufriedenstellende Versorgung des Sensors zur Erfassung des Atemalkoholgehalts ermöglicht.

Generell ist es denkbar, dass der Sensor zur Erfassung des Atemalkoholgehalts in einem Strömungskanal angeordnet ist, der derart ausgeführt ist, dass die Probe in diesen eingeleitet und in entgegengesetzter Richtung wieder ausgeleitet wird, ohne dass zusätzliche Hilfseinrichtungen vorgesehen sind. Ebenso kann eine Pumpeneinheit vorgesehen sein, die bspw. über einen Drucksensor angesteuert wird, und dem Sensor die Probe als Teilstrom eines an einem Abzweig vorbeigeleiteten oder anstehenden Gesamtvolumenstroms zuleitet. In diesem Fall wird auf vorteilhafte Weise die für die Testdurchführung erforderliche Menge von Atemluft bei Erreichen oder Überschreiten eines Druckgrenzwertes am Drucksensor aus dem Hauptströmungskanal in den Messgaskanal des Messluftzweigs abgezweigt. Sofern das erforderliche Probenvolumen mit Hilfe einer Pumpeinheit in den Messgaskanal hineingesogen wird, bietet das den Vorteil, dass stets eine reproduzierbare Messung und Auswertung der Atemprobe erfolgt.

Weiterhin ist es vorteilhaft, wenn die wenigstens eine zusätzliche Ausströmöffnung derart gestaltet und dimensioniert ist, dass die Erzeugung eines Messsignals durch den Sensor aufgrund von Umgebungseinflüssen, die nicht durch die Abgabe der Atemprobe hervorgerufen werden, verhindert wird. Insbesondere wenn eine Atemalkoholmessvorrichtung im Freien verwendet wird, kann sowohl der Feuchtegehalt als auch die Temperatur der Umgebung teilweise erheblich variieren. Diese Variation der Umgebungsbedingungen sollte für die Auswertung der Atemalkoholprobe des Probanden unerheblich sein. Auf bevorzugte Weise ist daher im Bereich des Probensammlers und/oder in Strömungsrichtung hinter dem Sammlerauslass des Probensammlers zumindest ein Heizelement vorgesehen, das zuverlässig Kondensationserscheinungen innerhalb der Atemalkoholmessvorrichtung, insbesondere innerhalb der Messeinheit, verhindert.

Gemäß einer weiteren speziellen Gestaltung der Erfindung verfügt das Rückhalteelemente über wenigstens ein Befestigungselement, das derart ausgeführt ist, dass das Rückhalteelement zerstörungsfrei mit dem Probensammler verbindbar und nach einem Verbinden wieder zerstörungsfrei lösbar ist. Auf vorteilhafte Weise ist es ferner denkbar, dass das Rückhalteelement mit dem Probeneinlass als einteiliges Bauteil ausgeführt ist, das wechselbar am Probensammler befestigt werden kann. Ebenso ist es denkbar, dass das Rückhalteelement als separates Bauteil ausgeführt, das im Bedarfsfall auf einfache Weise mit dem Probensammler, insbesondere durch eine Steck- und/oder Rastverbindungen, verbunden wird. Je nach gewählter Ausführungsform ist es bei Bedarf somit auf einfache Weise möglich, das Rückhalteelement mit oder ohne den Probeneinlass auszutauschen.

Auf vorteilhafte Weise verfügt die wenigstens eine zusätzlich Ausströmöffnung über wenigstens zwei in einer Außenwand des Probensammlers eingebrachte Schlitze. Die Schlitze sind hierbei derart ausgeführt, dass ein Teil des vom Probanden in den Probeneinlass des Probensammlers eingeleiteten Atemgasstromes aus dem Probensammler in die Umgebung ausströmt und es so zu einer gleichmäßigen Durchströmung des Probensammlers bis hin zum Sammelauslass kommt. Bevorzugt wird die Ausströmöffnung durch mehrere Öffnungen gebildet, die über den Umfang des Strömungskanals verteilt in eine Außenwand des Probensammlers eingebracht sind. Weiterhin ist es denkbar, dass sich ein Querschnitt des Hauptströmungskanals im Probensammler vom Probeneinlass bis zum Probenauslass verringert. Grundsätzlich ist es denkbar, dass eine derartige Verringerung des Strömungsquerschnittes kontinuierlich oder in einzelnen diskreten Stufen erfolgt. Besonders vorteilhaft ist es, wenn der Hauptströmungskanal des Probensammlers im Bereich des Sammlerauslasses zumindest in einer Ebene eine trapezförmige Querschnittsform aufweist. Hierbei ist es sinnvoll, wenn das Gegenstück der Messeinheit des Atemalkoholmessgerätes, die an den Sammlerauslass, insbesondere an den trapezförmig gestalteten Sammlerauslass, angeschlossen wird, zumindest einen ähnlichen Querschnitt aufweist. Ergänzend oder alternativ ist es ferner denkbar, dass im Bereich des Sammlerauslasses eine zumindest teilweise Umlenkung des Atemgasstromes erfolgt. In diesem Zusammenhang ist es beispielsweise denkbar, dass der Atemgasstrom aus einer Richtung in den Bereich des Sammlerauslasses einströmt, hier umgelenkt, in Teilströme aufgeteilt und über wenigstens zwei Öffnungen des Sammlerauslasses in die Messeinheit strömt. Gemäß dieser Ausführungsform wird der Hauptströmungskanal somit im Bereich des Sammlerauslasses zumindest kurzzeitig in zwei Teilströmungskanäle aufgeteilt, die sich im Anschluss an den Sammlerauslass wieder zu dem einen Hauptströmungskanal vereinigen. Durch die gezielte Umlenkung und/oder Aufteilung des Atemgasstroms im Bereich des Sammlerauslasses, ist es wiederum möglich, durch geeignete Dimensionierung der entsprechenden Strömungswiderstände besonders vorteilhafte Strömungsbedingungen im Probensammler und in der Messeinheit, insbesondere eine besonders geeignete Durchströmung des Hauptströmungskanals sowie Messgasversorgung des Sensors, sicherzustellen.

In einer speziellen Weiterbildung der Erfindung weist der Probensammler wenigstens ein Befestigungselement auf, sodass der Probensammler zerstörungsfrei und werkzeugfrei mit der Messeinheit, in der der Sensor angeordnet ist, verbindbar und nach dem Verbinden wieder zerstörungsfrei lösbar ist.

Weiterhin ist es denkbar, dass zwischen dem Probeneinlass des Probensammlers und dem Sensor im Bereich des Hauptströmungskanals wenigstens ein die Atemgasströmung beeinflussendes Bauelement, wie etwa ein Sieb, eine Membran, eine Klappe und/oder ein Ventil vorgesehen ist.

Im Folgenden wird die Erfindung ohne Beschränkung des allgemeinen Erfindungsgedankens anhand von Ausführungsbeispielen unter Bezugnahme auf die Figuren näher erläutert. Dabei zeigen:
- Figur 1: Front- und Seitenansicht eines erfindungsgemäß ausgeführten Probensammlers für ein Atemalkoholmessgerät, das eine kontaktlose Abgabe einer Atemprobe für einen Probanden ermöglicht;
- Figur 2: Schnittdarstellung eines erfindungsgemäß ausgeführten Probensammlers;
- Figur 3: perspektivische Ansicht eines erfindungsgemäß ausgeführten Probensammlers mit entnommenem Probeneinlass;
- Figur 4: Atemalkoholmessgerät mit Probensammler sowie
- Figur 5: Strömungsgrafik eines geschlossenen Trichtermundstücks sowie
- Figur 6: Strömungsgrafik eines Probensammlers mit erfindungsgemäß vorgesehenen Ausströmöffnungen.

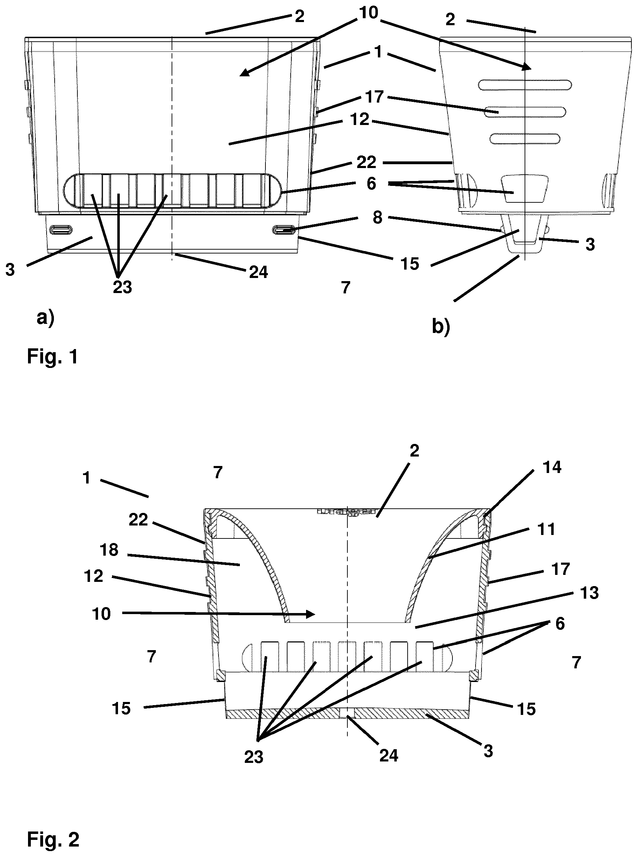

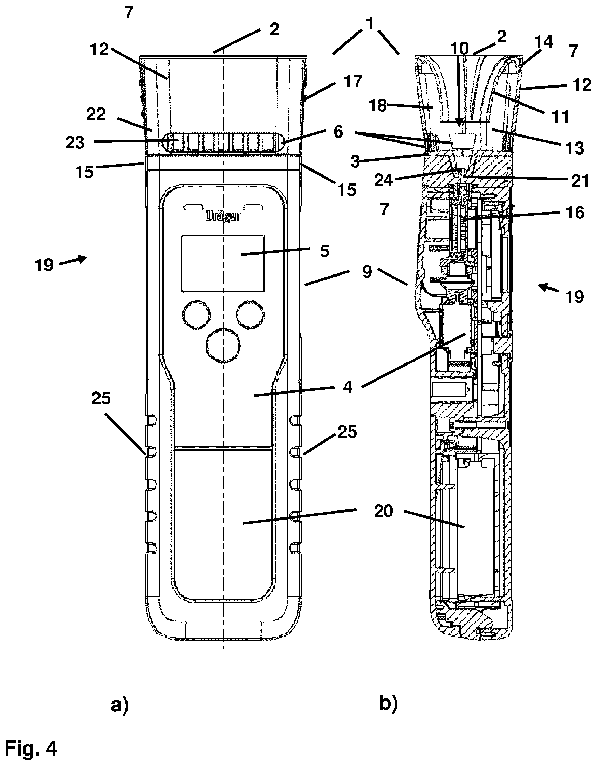

In Figur 1 ist in einer Frontansicht gemäß Figur 1a und einer Seitenansicht gemäß 1b ein erfindungsgemäß ausgeführter Probensammler 1 für ein erfindungsgemäß ausgeführtes Atemalkoholmessgerät 19 dargestellt. Das Atemalkoholmessgerät 19, das eine Messeinheit 9 und einen daran befestigten Probensammler 1 aufweist, ist Figur 4 gezeigt.

Der in zwei Ansichten dargestellte Probensammler 1 verfügt über einen Probeneinlass 2, in den ein Proband eine Atemprobe abgibt, ohne dass der Probeneinlass 2 bzw. der Probensammler 1 hierfür berührt werden muss. Vielmehr wird während der Probenabgabe die Atemprobe in den Probeneinlass 2 hineingepustet, ohne dass es zu einer Berührung des Probeneinlasses 2 kommt. Der Probensammler 1 ist trichterförmig ausgeführt, sodass die vom Probanden abgegebene Atemprobe in einen im Inneren des Probensammlers 1 angeordneten Hauptströmungskanal 10 hineingeleitet wird. Durch diesen Hauptströmungskanal 10 gelangt die Atemprobe vom Probeneinlass 2 zum Sammlerauslass 3 des Probensammlers 1.

Im Bereich des Sammlerauslass 3 ist ein spezielles Befestigungselement 8 vorgesehen, das eine Befestigung des Probensammlers 1 an der Messeinheit 9 des Atemalkoholmessgeräts 19, ermöglicht. In der Messeinheit 9 befinden sich insbesondere der Sensor 16 zur Detektion des Atemalkoholgehaltes, eine Steuer- und Auswerteeinheit 4, eine Energieversorgung 20, die bevorzugt über wenigstens einen aufladbaren Akkumulator verfügt, sowie eine Ausgabeeinheit 5, insbesondere in Form eines Displays. Das Befestigungselement 8 im Bereich des Sammlerauslass 3 ist derart ausgeführt, dass der Probensammler 1 zerstörungsfrei und ohne Zuhilfenahme eines Werkzeugs an der Messeinheit 9 des Atemalkoholmessgeräts 19 befestigt und von diesem wieder gelöst werden kann. Stromabwärts des Sammlerauslasses 3 setzt sich der Hauptströmungskanal 10 innerhalb der Messeinheit 9 fort, wobei der vom Probanden abgegebene Atemgasstrom zumindest zum Teil kontinuierlich über einen Messgaskanal 21 zum Sensor 16 geleitet wird, sodass der Sensor 16 bedarfsgerecht mit einem erforderlichen Probenvolumen versorgt wird.

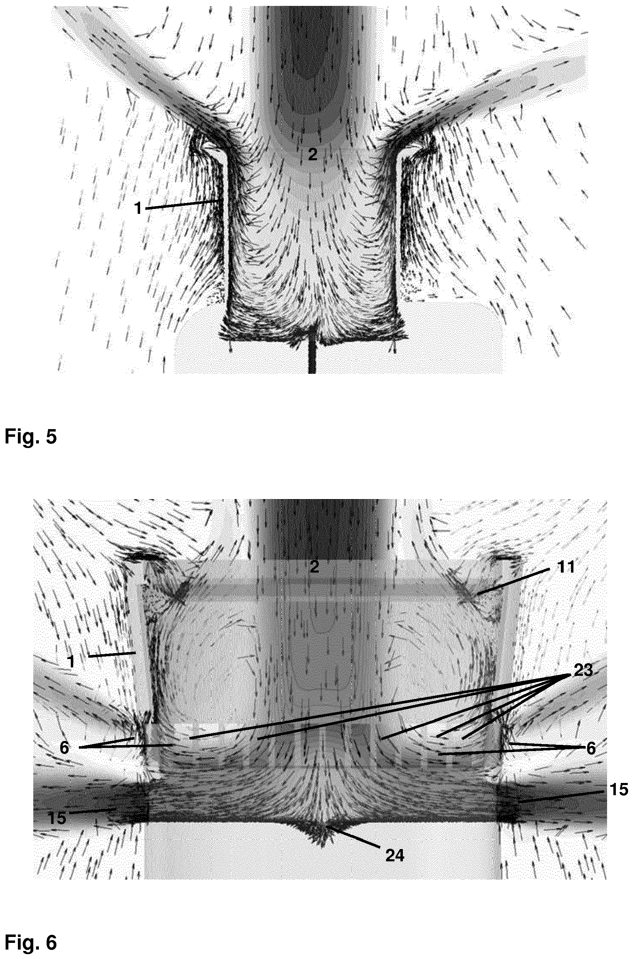

Erfindungswesentlich an dem in Figur 1 dargestellten Probensammler 1 ist, dass im unteren Bereich eine zusätzliche Ausströmöffnung 6, die eine Mehrzahl von nebeneinander angeordneten Schlitzen bzw. Durchbrüchen 23 aufweist, vorgesehen ist. Durch die in diesem Bereich vorgesehene Ausströmöffnung 6 mit den entsprechenden Schlitzen 23 gelangt zumindest ein Teil der in den Probeneinlass 2 eingeblasen Atemprobe in eine Umgebung 7 des Probensammlers 1. Dieser Teil der Atemprobe, der durch die Ausströmöffnung 6 in die Umgebung 7 gelangt, wird nicht der Messeinheit 9 mit dem Sensor 16 zugeführt, sondern gelangt in die Umgebung 7, bevor der restliche Teil der Atemprobe in den Sammlerauslass 3 einströmt. Aufgrund des Vorsehens von Ausströmöffnungen 6 in Form von Schlitzen 23 wird eine besonders geeignete Durchströmung des Hauptströmungskanals 10 im Inneren des Probensammlers 1 ermöglicht. Vor allem werden lokal erhöhte Staudrücke sowie Rückströmungen in Richtung auf den Probeneinlass 2 durch die vorgesehenen Ausströmöffnungen 6 zuverlässig verhindert. Ergänzend hierzu zeigt Figur 6 das Strömungsbild eines Probensammlers 1, der von einer Atemprobe durchströmt wird. Dieses Strömungsbild wird später noch näher erläutert werden.

In dem in Figur 1 gezeigten Ausführungsbeispiel verfügt der Sammlerauslass 3 über insgesamt drei Auslässe, über die der vom Probanden in den Probeneinlass 2 des Probensammlers 1 abgegebene Atemgasstrom aus dem Sammlerauslass 3 ausströmt. An den zwei seitlich angeordneten Auslässen 15 endet der Hauptströmungskanal 10 bzw. mündet in die Umgebung 7. Über eine an der Unterseite des Sammlerauslasses 3 und in Flucht mit dem Probeneinlass 2 befindliche Öffnung 24 gelangt ein Teil des vom Probanden abgegebenen Atemgasstroms in des Messgaskanal 21 der Messeinheit 9 und schließlich zum Sensor 16 zur Atemalkoholbestimmung. Nachdem die Messung abgeschlossen ist strömt das im Messgaskanal 21 befindliche Atemgas zurück in den Sammlerauslass 3 und schließlich über die seitlichen Austrittsöffnungen 24 in die Umgebung 7.

Alternativ ist es denkbar, dass der Messgaskanal über eine andere Öffnung, insbesondere einen in der Messeinheit 9 vorgesehenen Auslass entlüftet wird.

Figur 2 zeigt den erfindungsgemäß ausgeführten Probensammler 1 in einer Schnittansicht. Der Probensammler 1 ist trichterförmig ausgeführt und verfügt über eine Außenwand 12, an der nach außen gerichtete Vorsprünge 17 vorgesehen sind, die ein leichtes Ergreifen und Befestigen des Probensammlers 1 an einer Messeinheit 9 eines Atemalkoholmessgeräts 19 ermöglichen. Der gesamte Probensammler 1 ist aus einem Kunststoff hergestellt und daher besonders hygienisch, leicht zu reinigen und kann auf bevorzugte Weise als auswechselbares Einwegteil ausgeführt werden.

Im Bereich des Probeneinlasses 2 ist mittels geeigneter Befestigungselemente 14 ein trichterförmiges Rückhalteelement 11 an dem Probensammler 1 befestigt, das eine düsenartige Führung für den Atemgasstrom im Hauptströmungskanal 10 sicherstellt und ferner für Rückströmungen aus dem unteren Bereich des Probensammlers 1 in Richtung des Probeneinlasses 2 ein Strömungshindernis darstellt. Wesentlich hierbei ist, dass der Probeneinlass 2 und das Rückhalteelement 11 derart ausgeführt sind, dass die Atemprobe abgegeben wird, ohne dass der Proband den Probeneinlass 2 berühren muss. Die in den Probeneinlass 2 des Probensammlers 1 abgegebene Atemprobe durchströmt das konusartig bzw. düsenartig ausgeführte Rückhalteelement 11 durch den Hauptströmungskanal 10 in Richtung des Sammlerauslasses 3. Generell ist es denkbar dass der Probeneinlass 2 mit dem Rückhalteelement 11 als ein Bauteil oder als separate, mit einander durch Befestigungselemente 14 verbindbare Bauteile ausgeführt sind und dieses eine oder diese zwei Bauteile mit den übrigen Teilen des Probensammlers 1 verbunden werden. Ebenso ist es denkbar den Probensammler 1 mit Probeneinlass 2 und Rückhalteelement 11 als einteiliges Bauteil, insbesondere aus einem Kunststoff, herzustellen.

Die in Figur 2 gezeigte Ausführungsform des Rückhalteelementes 11 ermöglicht einerseits eine zuverlässige Zuführung der Atemprobe in das Innere des Probensammlers 1 und darüber hinaus wird ein Rückströmen zumindest eines Teils der Atemprobe in Richtung des Probeneinlasses 2 zuverlässig verhindert. Dies wird dadurch erreicht, dass das trichterförmig ausgeführte Rückhalteelement 11 in den Hauptströmungskanal 10 des Probensammlers 1 hineinragt und etwa die Hälfte der Querschnittsfläche abdeckt, sodass beidseitig des trichterförmigen Rückhalteelementes 11 zwischen dem Rückhalteelement 11 und der Außenwand 12 eine Totraumzone 18 gebildet wird, in der sich teilweise Atemgas ansammelt.

Wesentlich ist, dass in Strömungsrichtung der abgegebenen Atemprobe hinter dem Rückhalteelement 11 wiederum eine Ausströmöffnung 6 mit einer Mehrzahl von schlitzförmigen bzw. rechteckigen Durchbrüchen 23 vorgesehen ist, durch die ein Teil der in den Probensammler 1 abgegebenen Atemprobe in die Umgebung 7 entweicht. Stromabwärts dieser Ausströmöffnung 6 befindet sich schließlich der Sammlerauslass 3 der über drei Auslässe verfügt, wobei über eine Öffnung 24 an der Unterseite des Sammlerauslasses 3 ein Teil des Atemgases ausströmt und bevorzugt in einen Messgaskanal 21 einer Messeinheit 9 (hier nicht dargestellt) eines Atemalkoholmessgeräts 19 zur Messung des Atemalkoholgehalts geleitet wird. Darüber hinaus verfügt der Sammlerauslass 3 über zwei seitliche Auslässe 15, an denen der Hauptströmungskanal 10 in die Umgebung 7 mündet. Über diese Auslässe 15 strömt auf vorteilhafte Weise sowohl der Teil der Atemgasprobe, der nicht für die Atemalkoholmessung verwendet wurde, als auch der für die Atemalkoholmessung verwendete Teil nach der Messung in die Umgebung 7. Der Hauptströmungskanal 10 verfügt im Bereich des Sammlerauslasses 3 in Richtung auf die Auslässe 15 über einen trapezförmigen Querschnitt, wobei innerhalb des Sammlerauslasses 3 der nicht zur Öffnung 24 an der Unterseite geleitete Teil der Atemgasprobe um etwa 90° in Richtung auf die beiden seitlichen Auslässe umgelenkt wird.

Wesentlich für die Erfindung ist, dass der Probensammler 1 derart ausgeführt ist, dass eine gleichmäßige Durchströmung des Hauptströmungskanals 10 innerhalb des Probensammlers 1 gewährleistet ist. Die Ausströmöffnung 6 kann generell eine oder eine Mehrzahl von Öffnungen aufweisen und in Strömungsrichtung vor, nach oder im Bereich eines Einlasses eines Messgaskanals 21, der zum Sensor 16 der Messeinheit 9 führt, positioniert werden. Die Ausströmöffnung stellt somit zusätzlich zum Auslass 15 am Ende des Hauptströmungskanals 10 eine Verbindung zwischen dem Hauptströmungskanal 10 und der Umgebung 7 her. Generell ist es ebenfalls denkbar wenigsten zwei Ausströmöffnungen 6 vorzusehen, die in Strömungsrichtung hintereinander angeordnet sind. In jedem Fall ist die wenigstens eine zusätzliche Ausströmöffnung 6 derart gestaltet, dass durch die Einleitung einer Atemprobe in den Probeneinlass 2 des Probensammlers 1 und damit in den Hauptströmungskanal 10 eine Druckdifferenz zur Umgebung 7 am Sensor 16 und/oder an einem zusätzlich vorgesehenen Drucksensor (nicht dargestellt) zur Ansteuerung einer Pumpeneinheit, die den Sensor 16 mit der erforderlichen Atemluftprobe versorgt, aufgebaut wird, um so eine Messung am Sensor 16 auszulösen.

Weiterhin ist die Ausströmöffnung 6 derart gestaltet, dass mögliche Umgebungseinflüsse, wie sie etwa durch Wind oder spezielle Temperatur- und/oder Luftfeuchtwerte bzw. durch Schwankungen dieser Werte verursacht werden, und die ein selbstständiges Auslösen des Sensors verursachen können, ausgeschlossen sind. Die Ausströmöffnung 6 ist ferner derart angeordnet, dass der durch diese zusätzliche Ausströmöffnung 6 aus dem Hauptströmungskanal 10 ausströmende Teil der Atemprobe nicht durch eine Hand des Probanden behindert wird. Eine entsprechende Grifffläche des Atemalkoholmessgerätes 19, insbesondere im Bereich eines Gehäuse der Messeinheit 9, ist daher mit ausreichend Abstand zur Ausströmöffnung 6 angeordnet. Aufgrund des Vorsehens der Ausströmöffnung 6 wird der Probensammler besser durchspült als bekannte Mundstücke, sodass nicht nur Rückströmungen zum Probanden verhindert oder zumindest minimiert werden, sondern sich auch die Genauigkeit der Atemalkoholmessung erhöht.

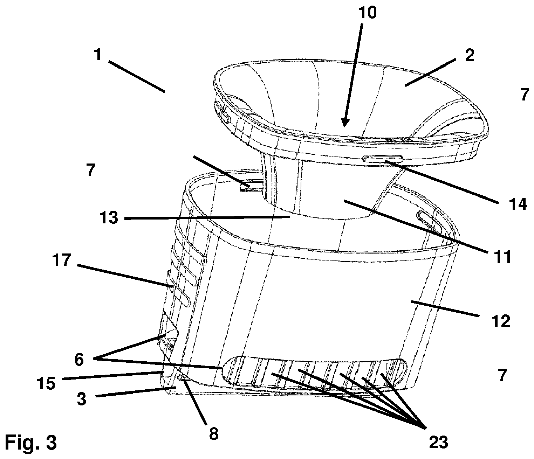

Figur 3 zeigt in einer perspektivischen Ansicht den erfindungsgemäß ausgeführten Probensammler 1, der einerseits ein Gehäuse 22 und andererseits ein Bauteil, das einen Probeneinlass 2 und ein Rückhalteelement 11 aufweist und das mit Hilfe von Befestigungselementen 14 am Gehäuse 22 befestigt werden kann. Das Bauteil, in dem der Probeneinlass 2 und das Rückhalteelement 11 integriert sind und das Gehäuse bilden gemäß dieser Ausführungsform gemeinsam den Probensammler 1.

In Figur 3 sind das Gehäuse 22 des Probensammlers 1 und der Probeneinlass 2 sowie das Rückhalteelement 11 in voneinander getrenntem Zustand dargestellt. Um das Bauteil, das den Probeneinlass 2 mit dem Rückhalteelement 11 aufweist, an dem Gehäuse 22 des Probensammlers 1 zu befestigen, wird dieses in den Probensammler 1 eingesteckt und die vier als Befestigungselemente 14 vorgesehenen Rastnasen rasten in entsprechende Ausnehmungen am Innenumfang des Gehäuses 22 ein. Um das Bauteil wiederum vom Gehäuse 22 des Probensammlers 1 zu entfernen, werden die Rastnasen, beispielsweise durch festes Ziehen des Bauteils oder durch zumindest teilweises Eindrücken wieder aus den entsprechenden Ausnehmungen entfernt.

Wesentlich an dem in Figur 3 gezeigten Probensammler 1 ist wiederum, dass im unteren Bereich eine Ausströmöffnung 6 mit mehreren rechteckigen Schlitzöffnungen 23 vorgesehen ist, durch die zumindest ein Teil der in den Probeneinlass 2 des Probensammlers 1 eingeblasenen Atemprobe aus dem Hauptströmungskanal 10 in die Umgebung 7 gelangt. Der übrige Teil der Atemprobe, der nicht durch die Ausströmöffnung 6 in die Umgebung 7 abgeleitet wurde, wird stromabwärts der Ausströmöffnung 6 zum einen Teil zur unteren Öffnung 24 des Sammlerauslasses 3, an die sich im Betriebszustand, in dem der Probensammler 1 mit einer Messeinheit 9 verbunden ist, ein Messgaskanal 21 anschließt, und zum anderen Teil umgelenkt und zu den seitlichen Auslassöffnungen 15 geleitet. Durch die Auslassöffnungen 15 des Sammlerauslasses 3, die in diesem Fall an zwei gegenüberliegenden Seiten des Sammlerauslasses 3 angeordnet sind, gelangt die abgegebene Atemgasprobe in die Umgebung 7. Innerhalb des Sammlerlauslass wird der Hauptströmungskanal 10 somit zumindest kurzzeitig in drei Teilkanäle aufgeteilt, nämlich zwei Teilströme, die über die seitlichen Auslassöffnungen 15 in die Umgebung gelangen, und einen Teilstrom, der über die untere Öffnung 24 in den Messgaskanal 21 einer Messeinheit 9 einleitbar ist.

An den äußeren Seitenwänden des Probensammlers 1, hier des Probensammlergehäuses 22, sind Elemente 17 in Form von kleinen Vorsprüngen vorgesehen, die ein leichtes Ergreifen und Befestigen des Probensammlers am Atemalkoholmessgerät für den Probanden ermöglichen.

Figur 4 zeigt in zwei Ansichten ein Atemalkoholmessgerät mit einem dafür vorgesehenen Probensammler 1. In Figur 4a ist die Messeinheit 9 des Atemalkoholmessgeräts mit dem daran befestigten Probensammler 1 in einer Frontansicht dargestellt. Ergänzend zeigt Figur 4b die Messeinheit 9 mit dem daran befestigten Probensammler in einer um 90° gedrehten Schnittansicht.

Die Messeinheit 9 verfügt im unteren Bereich über eine Greiffläche 25 mit geeigneten Ausnehmungen, die ein sicheres Ergreifen, Halten und Hantieren mit dem Alkoholmessgerät gewährleisten. Darüber hinaus befindet sich im Inneren der Messeinheit 9, eine Energieversorgung 20 mit einem Akkumulator, eine zentrale Steuer- und Auswerteeinheit 4 sowie eine Ausgabeeinheit 5 in Form eines Displays. Über die Ausgabeeinheit 5 werden insbesondere Informationen in Bezug auf den in einer untersuchten Atemprobe enthaltenen Atemalkoholgehalt ausgegeben.

Nachdem der Probensammler 1 an der Messeinheit 9 befestigt und das Gerät in den betriebszustand versetzt worden ist, können von Probanden abgegebene Atemproben auf ihren Alkoholgehalt untersucht werden. Wesentlich für das gezeigte Atemalkoholmessgerät ist, dass ein Proband eine Atemprobe abgeben kann, ohne dass es zu einem Kontakt zwischen dem Probensammler 1 bzw. seinem Probeneinlass 2 und dem Mund des Probanden kommt. Vielmehr wird die Atemprobe lediglich in den Probeneinlass hineingepustet.

Die in den Probeneinlass 2 des Probensammlers 1 abgegebene Atemprobe durchströmt den Probensammler 1, wobei ein Teil des Atemgasstroms über die Ausströmöffnung 6, die eine Mehrzahl von schlitzförmigen Öffnungen 23 bzw. Gehäusedurchbrüchen aufweist, in die Umgebung austritt. Der nicht ausgetretene Teil strömt in den Sammlerauslass 3 und hier teilweise durch die seitlichen Auslassöffnungen in die Umgebung 7 oder über die Öffnung 24 in den Messgaskanal 21, der sich innerhalb der Messeinheit 9 des Atemalkoholmessgeräts befindet. Über den Messgaskanal 21 gelangt der zu untersuchende Teil der abgegebenen Atemgasprobe zum Sensor 16, der in Abhängigkeit des Atemalkoholgehaltes ein Messsignal erzeugt und an eine zentrale Steuer- und Auswerteeinheit 4 überträgt. Basierend auf dem Messsignal ermittelt die Steuer- und Auswerteeinheit 4 einen Wert für den Alkoholgehalt der abgegebenen Probe, sodass eine entsprechende Information durch die als Display ausgeführte Ausgabeeinheit 5 bereitgestellt werden kann. Das im Messgaskanal 21 befindliche Atemgas strömt nach erfolgter Messung zurück in den Sammlerauslass 3 und hier über die seitlichen Auslässe 15 in die Umgebung 7.

Wesentliches technisches des in Figur 4 gezeigten Atemalkoholmessgerätes ist wiederum die Ausströmöffnung 6 mit einer Mehrzahl von schlitzförmigen Durchbrüchen 23 im Gehäuse 22 des Probensammlers 1. Diese Ausströmöffnung gewährleistet eine gute Durchströmung des Probensammlers 1, die auch bei zu verschiedenen Zeitpunkten durchgeführten Probenabgaben nahezu nicht variiert.

In den Figuren 5 und 6 wird der durch die Erfindung erzielte Vorteil, nämlich eine verbesserte Durchströmung des Hauptströmungskanals 10 und insbesondere des innerhalb des Probensammlers 1 angeordneten Teils des Hauptströmungskanals 10, verdeutlicht. Die Figuren 5 und 6 zeigen hierbei die Ergebnisse von Strömungssimulationen, die einerseits an einem Probensammler 1, der keine speziellen Ausströmöffnungen vorsieht und andererseits an einem erfindungsgemäß ausgeführten Probensammlern mit spezieller Ausströmöffnung 6 in Form einer Mehrzahl rechteckiger Schlitze 23 durchgeführt worden sind. Beide Figuren zeigen jeweils in einer Schnittdarstellung den Probensammler 1, wobei die Strömung bzw. Strömungsgeschwindigkeit der von einem Probanden abgegebenen Atemprobe im Inneren des Probensammlers 1 mithilfe von Pfeilen visualisiert wird. Die Pfeile repräsentieren hierbei die Richtung der Strömung in dem jeweiligen Bereich, wobei es in Bereichen, in denen die dargestellten Strömungspfeile besonders dicht aneinander liegen, zu einer besonders starken Durchströmung kommt und die Strömungsgeschwindigkeit in diesen Bereichen besonders hoch ist.

Figur 5 zeigt, wie einleitend bereits erwähnt worden ist, die Durchströmung eines Probensammlers, der nicht über die erfindungsgemäß vorgesehene Ausströmöffnung 6, die den Hauptströmungskanal 10 im Inneren des Probensammlers 1 mit der Umgebung 7 verbindet, verfügt. Die durchgeführte Simulation verdeutlicht, dass es sowohl umfangseitig am Probeneinlass 2 als auch in den unteren Ecken des Probensammlers 1 zu vergleichsweise hohen Staudrücken, Verwirbelungen bzw. hohen Strömungsgeschwindigkeiten kommt. Ferner ist zu erkennen, dass sich die Strömung zumindest teilweise, insbesondere im Bereich des Probeneinlasses 2 umkehrt, sodass der Proband im Gesicht eine Rückströmung der Atemprobe spüren würde. Dies kann einerseits zu Verfälschungen des Messergebnisses führen und andererseits wird dies regelmäßig von einem Probanden als unhygienisch empfunden.

Im Gegensatz zu einer Lösung ohne wenigstens eine zusätzliche Ausströmöffnung zeigt Figur 6 ein Strömungsbild eines erfindungsgemäß ausgeführten Probensammlers 1, das ebenfalls mithilfe einer Strömungssimulation gewonnen wurde. Wesentlich hierbei ist, dass im unteren Bereich des Probensammlers 1 eine Ausströmöffnung 1 mit mehreren rechteckigen Schlitzen 23 vorgesehen ist, durch die zumindest ein Teil der in den Probensammler 1 durch den Probeneinlass 2 abgegebenen Atemprobe in die Umgebung 7 austritt.

Deutlich zu erkennen ist einerseits, dass aufgrund der erfindungsgemäß vorgesehenen Ausströmöffnung 6 mit Schlitzen 23 eine gleichmäßige Durchströmung des Probensammlers 1, insbesondere in seinem trichterförmigen Bereich erzielt wird. Rückströmungen der Atemprobe, die zu einer Anströmung des Gesichtes des Probanden führen würden, werden nahezu ausgeschlossen. Weiterhin stellt die spezielle Ausführung des Sammlerauslasses 3, der einerseits eine kleine mittige Ausströmöffnung 6 und weiterhin zwei zu beiden Seiten des Probensammlers 1 vorgesehene Öffnungen mit trapezförmigem Querschnitt verfügt zu einer Umlenkung der Strömung im Hauptströmungskanal 10, sodass im oberen, trichterförmigen Teil des Probensammlers 1 vergleichsweise große Wirbel erzeugt werden, die auf bevorzugte Weise die zentral durch den Probeneinlass 2 in den Hauptströmungskanal 10 einströmende Atemprobe einhüllen. Weiterhin führt die Umlenkung der Strömung im Bereich des Sammlerauslasses 3 zu einer gleichförmigen Durchströmung und zu hohen Strömungsgeschwindigkeiten im Bereich der seitlichen Auslässe 15 des Sammlerauslasses 3, sodass hier auf geeignete Weise ein Sog erzeugt wird. Durch die vergleichsweise kleine Öffnung 24 am Boden des Sammlerauslasse strömt ein Teil der Atemprobe, der einer Messung des Atemalkoholgehalts in einer Messeinheit 9 zuführbar ist, aus. Auch die Öffnung 24 ist derart gestaltet, dass aufgrund des vergleichsweise kleinen Strömungsquerschnitts eine hohe Strömungsgeschwindigkeit und damit Durchströmung im Bereich der Öffnung 24 erreicht wird.

Figur 6 zeigt somit deutlich, dass einerseits Rückströmungen oder Totraumzonen innerhalb des Probeneinlasses 2, die vor allem aus hygienischer Sicht nachteilig sind, zumindest nahezu verhindert werden und andererseits eine gleichmäßige Durchströmung des Probensammlers 1 sichergestellt ist.

Durch die erfindungsgemäß vorgesehene, wenigstens eine zusätzliche Ausströmöffnung 6, bevorzugt mit einer Mehrzahl von Schlitzen 23, wird somit eine gleichmäßige Durchströmung des Probensammlers 1 ermöglicht und gleichzeitig eine geeignete Versorgung des zur Ermittlung des Atemalkohols vorgesehenen Sensors 16 in der strömungstechnisch nachgeschalteten Messeinheit 9 eines Atemalkoholmessgeräts realisiert.

### BEZUGSZEICHENLISTE

- 1: Probensammler
- 2: Probeneinlass
- 3: Sammlerauslass
- 4: Steuer- und Auswerteeinheit
- 5: Ausgabeeinheit
- 6: Ausströmöffnung
- 7: Umgebung
- 8: Befestigungselement Probensammler/Messeinheit des Atemalkoholmessgeräts
- 9: Messeinheit des Atemalkoholmessgeräts
- 10: Hauptströmungskanal
- 11: Rückhalteelement
- 12: Außenwand
- 13: sprunghafte Querschnittsänderung
- 14: Befestigungselement Rückhalteelement / Probensammler
- 15: Auslass am Ende des Strömungskanals
- 16: Sensor
- 17: Greifelemente
- 18: Totraumzone
- 19: Atemalkoholmessgerät
- 20: Energieversorgung
- 21: Messgaskanal
- 22: Gehäuse des Probensammlers
- 23: schlitzförmige Durchbrüche
- 24: Öffnung an der Unterseite des Sammlerauslasses
- 25: Greiffläche der Messeinheit

## Patentansprüche

1. Vorrichtung zur Atemalkoholmessung mit einem Probensammler (1),
in den vom Probanden über einen Probeneinlass (2) eine Atemprobe einleitbar ist, mit einem in einer Messeinheit (9) der Vorrichtung angeordneten Sensor (16), dem ein Teil der in den Probeneinlass (2) abgegebenen Atemprobe zuführbar ist und wobei die Messeinheit (9) dazu ausgestaltet ist, auf der Grundlage eines in der Atemprobe enthaltenen Alkoholgehaltes ein Messsignal zu erzeugen,
mit einem Sammlerauslass (3), über den die in den Probensammler (1) abgegebene Atemprobe in die Messeinheit (9), in der der Sensor (16) angeordnet ist, einleitbar wird, und
mit einer Steuer- und Auswerteeinheit (4), die dazu ausgestaltet ist, unter Zugrundelegung des Messsignals den Alkoholgehalt der Atemprobe zu ermitteln und ein ergebnisspezifisches Signal an eine Ausgabeeinheit (5) der Vorrichtung zu übertragen, wobei die Ausgabeeinheit (5) dazu ausgestaltet ist, eine Information über den Atemalkoholgehalt der Atemprobe bereitzustellen,
wobei der Probensammler (1) und der Probeneinlass (2) zur kontaktlosen Einleitung der Atemprobe geeignet ausgeführt sind,
wobei an den Probeneinlass (2) ein im Inneren des Probensammlers (1) verlaufender Hauptströmungskanal (10) anschließt,
wobei die Vorrichtung dazu ausgestaltet ist, aus dem Hauptströmungskanal (10) den Sensor (16) über einen Messgaskanal (21) wenigstens zeitweise mit einem Teil der in den Probeneinlass (2) abgegebenen Atemprobe zu beaufschlagen, wobei der Hauptströmungskanal (10) in Strömungsrichtung hinter dem Messgaskanal (21) über einen Auslass (15) in eine Umgebung (7) mündet,
wobei zwischen dem Probeneinlass (2) und dem Auslass (15) des Hauptströmungskanals (10) wenigstens eine weitere Ausströmöffnung (6) vorgesehen ist, wobei die Vorrichtung dergestalt ausgestaltet ist, dass durch die wenigstens eine weitere Ausströmöffnung (6) ein Teil der in den Probeneinlass (2) eingeleiteten Atemprobe in eine Umgebung (7) austritt,
wobei sich die wenigstens eine weitere Ausströmöffnung (6) zwischen dem Probeneinlass (2) und dem Sammlerauslass (3) befindet **dadurch gekennzeichnet, dass**
der Probensammler (1) wenigstens ein nach innen in den Hauptströmungskanal (10) ragendes trichterförmiges Rückhalteelement (11) aufweist, das eine düsenartige Führung für den Atemgasstrom im Hauptströmungskanal (10) sicherstellt und dazu ausgestaltet ist, ein Rückströmen der Atemprobe in Richtung des Probeneinlasses (2) zumindest teilweise zu behindern.

2. Vorrichtung nach Anspruch 1,
**dadurch gekennzeichnet, dass** ein Querschnitt des Hauptströmungskanals (10) in Strömungsrichtung der abgegebenen Atemprobe zumindest abschnittsweise kleiner wird.

3. Vorrichtung nach einem der Ansprüche 1 bis 2,
**dadurch gekennzeichnet, dass** der Probensammler (1) wenigstens ein Befestigungselement (8) aufweist, durch das der Probensammler (1) mit der Messeinheit (9), in dem der Sensor (16) angeordnet ist, verbindbar ist, wobei sich der Hauptströmungskanal (10) in der Messeinheit (9) fortsetzt und in der Messeinheit (9) ein Abzweig vorgesehen ist.

4. Vorrichtung nach einem der Ansprüche 1 bis 3,
**dadurch gekennzeichnet, dass** die wenigstens eine weitere Ausströmöffnung (6) derart gestaltet und dimensioniert ist, dass nach Einleitung der Atemprobe in den Probensammler (1) am Sensor (16) oder einem Drucksensor, dessen Messsignal einer Ansteuerung einer Pumpeneinheit zur Versorgung des Sensors (16) mit dem zumindest einen Teil der Atemprobe zugrunde gelegt wird, ein Druck erzeugt wird, der eine Differenz zum Druck in der Umgebung (7) aufweist.

5. Vorrichtung nach einem der Ansprüche 1 bis 4,
**dadurch gekennzeichnet, dass** die wenigstens eine weitere Ausströmöffnung (6) derart gestaltet und dimensioniert ist, dass die Erzeugung eines Messsignals durch den Sensor (16) aufgrund von Umgebungseinflüssen, die nicht durch die Abgabe der Atemprobe hervorgerufen werden, verhindert wird.

6. Vorrichtung nach einem der Ansprüche 1 bis 5,
**dadurch gekennzeichnet, dass** das Rückhalteelement (11) im Bereich des Probeneinlasses (2) als den Hauptströmungskanal (10) umschließende, von einer Innenwand des Probensammlers (1) abstehende Trennwand ausgeführt ist.

7. Vorrichtung nach Anspruch einem der Ansprüche 1 bis 6,
**dadurch gekennzeichnet, dass** das Rückhalteelement (11) wenigstens ein Befestigungselement (14) aufweist, das derart ausgeführt ist, dass das Rückhalteelement zerstörungsfrei mit dem Probensammler (1) verbindbar und nach einem Verbinden wieder zerstörungsfrei lösbar ist.

8. Vorrichtung nach einem der Ansprüche 1 bis 7,
**dadurch gekennzeichnet, dass** die wenigstens eine weitere Ausströmöffnung (6) zumindest zwei in eine Außenwand (12) des Probensammlers (1) eingebrachte Schlitze (23) aufweist.

9. Vorrichtung nach einem der Ansprüche 1 bis 8,
**dadurch gekennzeichnet, dass** die wenigstens eine weitere Ausströmöffnung (6) durch mehrere Öffnungen gebildet wird, die über den Umfang des Strömungskanals (10) verteilt in eine Außenwand des Probensammlers (1) eingebracht sind.

10. Vorrichtung nach einem der Ansprüche 1 bis 9,
**dadurch gekennzeichnet, dass** sich ein Querschnitt des Hauptströmungskanals (10) im Probensammler (1) vom Probeneinlass (2) bis zu einem Sammlerauslass (3) verringert.

11. Vorrichtung nach einem der Ansprüche 1 bis 9,
**dadurch gekennzeichnet, dass** zwischen der wenigstens einen weiteren Ausströmöffnung (6) und dem Sammlerauslass (3) eine sprunghafte Verringerung (13) eines Querschnitts des Hauptströmungskanals (10) vorgesehen ist.

12. Vorrichtung nach einem der Ansprüche 1 bis 11,
**dadurch gekennzeichnet, dass** der Hauptströmungskanal (10) des Probensammlers (1) im Bereich des Sammlerauslasses (3) zumindest in einer Ebene eine trapezförmige Querschnittsform aufweist.

13. Vorrichtung nach einem der Ansprüche 1 bis 12,
**dadurch gekennzeichnet, dass** zwischen dem Probeneinlass (2) und dem Sensor (16) wenigstens ein strömungsbeeinflussendes Bauelement, insbesondere ein Sieb, eine Membran, eine Klappe und/oder ein Ventil angeordnet ist.

## Claims

1. Device for measurement of breath alcohol, with a sample collector (1) into which a breath sample can be introduced by the subject via a sample inlet (2), with a sensor (16) which is arranged in a measuring unit (9) of the device and to which part of the breath sample discharged into the sample inlet (2) can be delivered, and wherein the measuring unit (9) is designed to generate a measurement signal on the basis of an alcohol content contained in the breath sample, with a collector outlet (3) via which the breath sample discharged into the sample collector (1) is introducible into the measuring unit (9) in which the sensor (16) is arranged, and
with a control and evaluation unit (4) which is designed to determine the alcohol content of the breath sample on the basis of the measurement signal and to transmit a result-specific signal to an output unit (5), wherein the output unit (5) is designed to provide information concerning the breath alcohol content of the breath sample,
wherein the sample collector (1) and the sample inlet (2) are designed to be suitable for contactless introduction of the breath sample,
wherein a main flow channel (10) extending in the interior of the sample collector (1) adjoins the sample inlet (2),
wherein the device is designed such that, from the main flow channel (10), part of the breath sample discharged into the sample inlet (2) acts at least temporarily on the sensor (16) via a measuring gas channel (21), wherein the main flow channel (10), behind the measuring gas channel (21) in the direction of flow, opens into the environment (7) via an outlet (15), wherein at least one further outflow opening (6) is provided between the sample inlet (2) and the outlet (15) of the main flow channel (10), wherein the device is designed in such a way that part of the breath sample introduced into the sample inlet (2) emerges through the at least one further outflow opening (6) into the environment (7),
wherein the at least one further outflow opening (6) is located between the sample inlet (2) and the collector outlet (3), **characterized in that** the sample collector (1) has at least one funnel-shaped retaining element (11) which protrudes inwardly into the main flow channel (10) and which ensures a nozzle-like guide for the respiratory gas stream in the main flow channel (10) and is designed to at least partially impede a backflow of the breath sample in the direction of the sample inlet (2).

2. Device according to Claim 1, **characterized in that** a cross section of the main flow channel (10) becomes smaller in at least some sections in the direction of flow of the discharged breath sample.

3. Device according to either of Claims 1 and 2, **characterized in that** the sample collector (1) has at least one fastening element (8), by which the sample collector (1) is connectable to the measuring unit (9) in which the sensor (16) is arranged, wherein the main flow channel (10) continues in the measuring unit (9) and a branch-off is provided in the measuring unit (9).

4. Device according to one of Claims 1 to 3, **characterized in that** the at least one further outflow opening (6) is shaped and dimensioned in such a way that, after the breath sample has been introduced into the sample collector (1), a pressure different from the pressure in the environment (7) is generated at the sensor (16) or at a pressure sensor whose measurement signal serves as a basis for actuating a pump unit to supply the sensor (16) with the at least one part of the breath sample.

5. Device according to one of Claims 1 to 4, **characterized in that** the at least one further outflow opening (6) is shaped and dimensioned in such a way as to prevent the generation of a measurement signal by the sensor (16) on account of environmental influences that are not caused by the discharge of the breath sample.

6. Device according to one of Claims 1 to 5, **characterized in that** the retaining element (11) in the region of the sample inlet (2) is designed as a partition enclosing the main flow channel (10) and protruding from an inner wall of the sample collector (1).

7. Device according to one of Claims 1 to 6, **characterized in that** the retaining element (11) has at least one fastening element (14) which is designed in such a way that the retaining element is connectable to the sample collector (1) non-destructively and, after connection, is detachable again non-destructively.

8. Device according to one of Claims 1 to 7, **characterized in that** the at least one further outflow opening (6) has at least two slits (23) introduced into an outer wall (12) of the sample collector (1).

9. Device according to one of Claims 1 to 8, **characterized in that** the at least one further outflow opening (6) is formed by a plurality of openings which, distributed about the circumference of the flow channel (10), are introduced into an outer wall of the sample collector (1).

10. Device according to one of Claims 1 to 9, **characterized in that** a cross section of the main flow channel (10) in the sample collector (1) decreases from the sample inlet (2) to a collector outlet (3).

11. Device according to one of Claims 1 to 9, **characterized in that** an abrupt reduction (13) of a cross section of the main flow channel (10) is provided between the at least one further outflow opening (6) and the collector outlet (3).

12. Device according to one of Claims 1 to 11, **characterized in that** the main flow channel (10) of the sample collector (1) has a trapezoidal cross-sectional shape at least in one plane in the region of the collector outlet (3).

13. Device according to one of Claims 1 to 12, **characterized in that** at least one flow-influencing component, in particular a screen, a diaphragm, a flap and/or a valve, is arranged between the sample inlet (2) and the sensor (16).

## Revendications

1. Dispositif de mesure du taux d'alcool dans l'air expiré, ledit dispositif comprenant
un collecteur d'échantillons (1) dans lequel un échantillon d'air expiré peut être introduit par le sujet par une entrée d'échantillon (2),
un capteur (16) qui est disposé dans une unité de mesure (9) du dispositif et auquel peut être amenée une partie de l'échantillon d'air expiré qui a été délivrée dans l'entrée d'échantillon (2) et l'unité de mesure (9) étant conçue pour générer un signal de mesure sur la base d'une teneur en alcool contenue dans l'échantillon d'air expiré,
une sortie de collecteur (3) par laquelle l'échantillon d'air expiré qui a été délivrée dans le collecteur d'échantillons (1) peut être introduit dans l'unité de mesure (9) dans laquelle est disposé le capteur (16), et
une unité de commande et d'évaluation (4) qui est conçue pour déterminer la teneur en alcool de l'échantillon d'air expiré sur la base du signal de mesure et pour transmettre un signal spécifique au résultat à une unité de sortie (5) du dispositif, l'unité de sortie (5) étant conçue pour fournir une information sur la teneur en alcool dans l'échantillon d'air expiré,
le collecteur d'échantillons (1) et l'entrée d'échantillon (2) étant conçus de manière appropriée à l'introduction sans contact de l'échantillon d'air expiré,
un canal d'écoulement principal (10) qui s'étend à l'intérieur du collecteur d'échantillons (1) étant relié à l'entrée d'échantillon (2),
le dispositif étant conçu pour soumettre, à partir du canal d'écoulement principal (10), le capteur (16) par le biais d'un canal de gaz de mesure (21) au moins temporairement à une partie de l'échantillon d'air expiré qui a été délivré dans l'entrée d'échantillon (2), le canal d'écoulement principal (10) débouchant dans un environnement (7) par une sortie (15) en aval du canal de gaz de mesure (21) dans la direction d'écoulement,
au moins une autre ouverture de sortie (6) étant prévue entre l'entrée d'échantillon (2) et la sortie (15) du canal d'écoulement principal (10), le dispositif étant conçu de manière à ce qu'une partie de l'échantillon d'air expiré, introduit à l'entrée d'échantillon (2), sorte dans un environnement (7) par l'au moins une autre ouverture de sortie (6),
l'au moins une autre ouverture de sortie (6) étant située entre l'entrée d'échantillon (2) et la sortie de collecteur (3), **caractérisé en ce que**
le collecteur d'échantillons (1) comporte au moins un élément de retenue (11) en forme d'entonnoir qui fait saillie vers l'intérieur dans le canal d'écoulement principal (10) et qui assure un guidage en forme de buse pour le flux de gaz d'air expiré dans le canal d'écoulement principal (10) et est conçu pour empêcher au moins partiellement l'échantillon d'air expiré de refluer en direction de l'entrée d'échantillon (2).

2. Dispositif selon la revendication 1, **caractérisé en ce qu'**une section transversale du canal d'écoulement principal (10) dans le sens d'écoulement de l'échantillon d'air expiré délivré devient par endroits plus petite.

3. Dispositif selon l'une des revendications 1 et 2, **caractérisé en ce que** le collecteur d'échantillons (1) comporte au moins un élément de fixation (8) permettant de relier le collecteur d'échantillons (1) à l'unité de mesure (9) dans laquelle le capteur (16) est disposé, le canal d'écoulement principal (10) continuant dans l'unité de mesure (9) et une dérivation étant prévue dans l'unité de mesure (9).

4. Dispositif selon l'une des revendications 1 à 3, **caractérisé en ce que** l'au moins une autre ouverture d'écoulement (6) est conçue et dimensionnée de manière à ce qu'une pression, qui présente une différence avec la pression dans l'environnement (7), est générée après l'introduction de l'échantillon d'air expiré dans le collecteur d'échantillons (1) sur le capteur (16) ou un capteur de pression, dont le signal de mesure est à la base d'une commande d'une unité de pompe destinée à alimenter le capteur (16) avec l'au moins une partie de l'échantillon d'air expiré.

5. Dispositif selon l'une des revendications 1 à 4, **caractérisé en ce que** l'au moins une autre ouverture d'écoulement (6) est conçue et dimensionnée de manière à empêcher la génération d'un signal de mesure par le capteur (16) en raison d'influences environnementales qui ne sont pas causées par la délivrance de l'échantillon d'air expiré.

6. Dispositif selon l'une des revendications 1 à 5, **caractérisé en ce que** l'élément de retenue (11) dans la région de l'entrée d'échantillon (2) est conçu comme une paroi de séparation entourant le canal d'écoulement principal (10) et saillant d'une paroi intérieure du collecteur d'échantillons (1).

7. Dispositif selon l'une des revendications 1 à 6, **caractérisé en ce que** l'élément de retenue (11) comporte au moins un élément de fixation (14) qui est conçu de manière à ce que l'élément de retenue puisse être relié de manière non destructive au collecteur d'échantillons (1) et puisse être détaché également de manière non destructive après une liaison.

8. Dispositif selon l'une des revendications 1 à 7, **caractérisé en ce que** l'au moins une autre ouverture de sortie (6) comporte au moins deux fentes (23) introduites dans une paroi extérieure (12) du collecteur d'échantillons (1).

9. Dispositif selon l'une des revendications 1 à 8, **caractérisé en ce que** l'au moins une autre ouverture de sortie (6) est formée par une pluralité d'ouvertures qui sont ménagées de manière répartie sur la circonférence du canal d'écoulement (10) dans une paroi extérieure du collecteur d'échantillons (1).

10. Dispositif selon l'une des revendications 1 à 9, **caractérisé en ce qu'**une section transversale du canal d'écoulement principal (10) dans le collecteur d'échantillons (1) décroît depuis l'entrée d'échantillon (2) jusqu'à une sortie de collecteur (3).

11. Dispositif selon l'une des revendications 1 à 9, **caractérisé en ce qu'**une réduction brutale (13) d'une section transversale du canal d'écoulement principal (10) est prévue entre l'au moins une autre ouverture d'écoulement de sortie (6) et la sortie de collecteur (3).

12. Dispositif selon l'une des revendications 1 à 11, **caractérisé en ce que** le canal d'écoulement principal (10) du collecteur d'échantillons (1) dans la région de la sortie de collecteur (3) a une section transversale de forme trapézoïdale au moins dans un plan.

13. Dispositif selon l'une des revendications 1 à 12, **caractérisé en ce qu'**au moins un composant influant sur l'écoulement, notamment un tamis, une membrane, un volet et/ou une soupape, est disposé entre l'entrée d'échantillon (2) et le capteur (16).
